# EUROPEAN PATENT SPECIFICATION

(11) **EP 0 782 504 B1**
(45) Date of publication and mention of the grant of the patent: **19.01.2000**
(21) Application number: 95933152.1
(22) Date of filing: 20.09.1995
(51) Int. Cl.: B32B 27/12, A61B 19/08

(54) **MICROPOROUS FILM/NONWOVEN COMPOSITES**
VERBUNDSTOFFE AUS MICROPORÖSEM FILM UND VLIESSTOFF
MATERIAU COMPOSITES EN FILM MICROPOREUX/NON-TISSE

(30) Priority: 20.09.1994 US 309841
(43) Date of publication of application: 09.07.1997
(73) Proprietor: THE UNIVERSITY OF TENNESSEE RESEARCH CORPORATION, Knoxville, TN 37996-1527 (US)
(72) Inventor: WADSWORTH, Larry, C., Knoxville, TN 37922 (US); GOSAVI, Nataraj, Knoxville, TN 34919 (US); KHAN, Ahamad, Yacub, Knoxville, TN 37909 (US)
(74) Representative: McCall, John Douglas
(86) International application number: US9511865
(87) International publication number: WO9609165

(56) References cited:
- EP-A- 0 006 264
- EP-A- 0 045 137
- WO-A-93/08024
- US-A- 4 632 860
- DATABASE WPI Section Ch, Week 8802 Derwent Publications Ltd., London, GB; Class A17, AN 88-009323 & JP,A,62 270 639 ( TOYO SODA MFG KK) , 25 November 1987

## Description

### BACKGROUND OF THE INVENTION

This invention relates generally to microporous/nonwoven composites which exhibit breathability and good barrier properties. In one aspect it relates to a composite comprising a nonwoven layer, a microporous layer, and a staple fiber layer. In another aspect, the invention relates to a method of laminating webs comprising a nonwoven web, a microporous web, and a staple fiber web.

Microporous films (or membranes as they are frequently referred to) have long been used in applications requiring both breathability (or water vapor transmissibility) and barrier to liquids. Commercially available microporous films include Celgard 2400 polypropylene film, EXXAIRE polyethylene film produced by Exxon Chemical Company, and TetraTex, a microporous polytetrafluoroethylene film produced by TetraTek Corporation, and Gore-Tex produced by W. L. Gore & Associates. Other microporous films include those made from polyamides, polyesters, polyurethane, and polypropylene.

Nonwoven webs, because of their high porosity, are highly breathable but exhibit little or no barrier properties. Efforts have been made to combine nonwovens with microporous film to arrive at a composite which possesses a balance of breathable and barrier properties. Such a composite has uses in health care, protective apparel, footwear, etc.

A paper entitled "EXXAIRE PLUS NON-WOVENS - MADE FOR EACH OTHER" was presented at the First Annual TANDEC Conference during October 22-25, 1991 in Knoxville, Tennessee. This paper disclosed a two layer composite comprising a microporous film and a nonwoven HDPE.

An article appearing in Nonwovens Industry dated June 1991, page 38, and entitled "New Light-weight Film Creating Markets for Nonwoven Composites" discloses a no-heat process for laminating a microporous film to nonwovens using discrete bonding patterns of an adhesive.

Patents which disclose microporous films and microporous film composites include the following:
(a) U.S. Patent 4,777,073 discloses a breathable polyolefin film prepared by melt embossing a highly filled polyolefin film which is stretched to impart greater permeability to the film;
(b) U.S. Patent 4,929,303 discloses a breathable polyolefin film heat laminated to a nonwoven HDPE fabric.

Nonwoven composites are described in the following U.S. Patents:
(a) U.S. Patent 4,929,303 discloses a composite comprising breathable polyolefin microporous films and nonwoven fabrics.
(b) U.S. Patent 4,041,203 discloses a composite of continuous filaments mat thermally bonded to a web of discontinuous filaments.
(c) U.S. Patent 4,142,016 discloses a multi-layered structure having a layer of textile fibers and a layer of staple short fibers bound together by a bonding agent.
(d) U.S. Patent 4,194,939 discloses a composite comprising layers of bulk fibers bonded between two reinforcing textile layers.
(e) U.S. Patent 4,675,226 discloses an inner layer of cellulose fibers and outer layers of a continuous filament thermoplastic meltblown fibers or rayon fibers. The layers are stitch-bonded together.
(f) U.S. Patent 4,950,531 discloses a composite of a meltblown fiber layer and a nonwoven material such as pulp fibers, staple fibers, meltblown fibers, and continuous filaments. The layers are hydraulically entangled together.
(g) U.S. Patent 4,970,104 discloses at least two non-woven webs bonded together by entangled bonding in spots by jet treatment.
(h) U.S. Patent 5,149,576 discloses a composite structure comprising nonwoven webs joined together by a mixture of an additive and a thermoplastic polymer.
(i) U.S. Patent 5,178,931 discloses a composite structure comprising three nonwoven layers of different filament diameters. The boundary between adjacent nonwoven layers is treated with an agent and the three-layered structure is bonded by the application of heat and pressure.
(j) U.S. Patent 5,200,246 discloses a composite comprising continuous longitudinally extending fibers bonded to webs of nonwovens such as spunbonded webs, meltblown webs, air laid webs, hydroentangled webs, film spun laced webs, etc. The webs are bonded together by meltblowing adhesives at the interfaces.
(k) U.S. Patent 5,230,949 discloses microporous fibers and filaments that can be used alone or in combination with other materials to form nonwoven web laminates.
(1) U.S. Patent 5,236,771 discloses a composite lining fabric for apparel use comprising a layer of melt-blown fibers bonded to a nonwoven layer of staple fibers or filaments (point bonded and fluid jet entangled).
(m) PCT Application PCT/US93/01783 discloses a multi-layered nonwoven composite comprising a layer of meltblown fibers, a layer of spunbonded fibers, and a layer of staple fibers such as cellulosic based fibers thermally bonded together.

### SUMMARY OF THE INVENTION

The present invention relates to composite web structures which are breathable and possess good strength and barrier properties. In addition, the composite constructed according to the present invention exhibits desirable aesthetic and comfort properties.

Although the composite of the present invention has a wide range of uses where breathability and barrier properties are necessary, it is particularly adapted for use as protective apparel.

The composite of the present invention comprises, in its broadest embodiment, a three-layered structure having a core layer of staple fiber web, flanked by, and adhesively bonded to, a microporous film, and a nonwoven web.

In a preferred embodiment of the invention, the composite comprises the following four layers:
(a) a first nonwoven web, preferably a meltblown or spundbond web;
(b) a microporous film adhesively bonded to the nonwoven web (a);
(c) a staple fiber web, preferably cotton staple fibers, adhesively bonded to the microporous film; and
(d) a second nonwoven web, preferably a meltblown web, adhesively bonded to the staple fiber web.

The structure combines the barrier properties of the microporous film, the breathability and strength of the non-woven webs, and the comfort and wicking properties of the staple fibers. The nonwoven webs also improve the aesthetics (appearance and soft hand) and add to the comfort for the user. Tests have also shown that the nonwoven webs, particularly meltblown webs, contribute to the barrier properties of the composite. The staple fiber web which exhibits hydrophilic characteristics, provides a wicking or reservoir layer for moisture and aqueous liquids.

The preferred method of laminating the webs to form the composite involves the steps of (a) applying a thin coat of an adhesive onto the webs so that the adhesive is at the interface of each web to form a composite and (b) feeding the composite into the nip of calender rolls maintained at a low temperature (e.g. less than 100°C) to pressure bond the webs together. The method may be carried out in one pass through the calender wherein all four layers are pressure bonded together, or in two or more passes through the calender wherein two or three layers are bonded together in one pass followed by the addition of one or more layers in subsequent passes.

A particularly surprising aspect of the present invention is that it produces composite structures that are capable of passing both the Blood and Viral Penetration Tests for protective clothing materials under ASTM Designations ES 21-92 and ES 22-92, respectively.

### BRIEF DESCRIPTION OF THE DRAWINGS

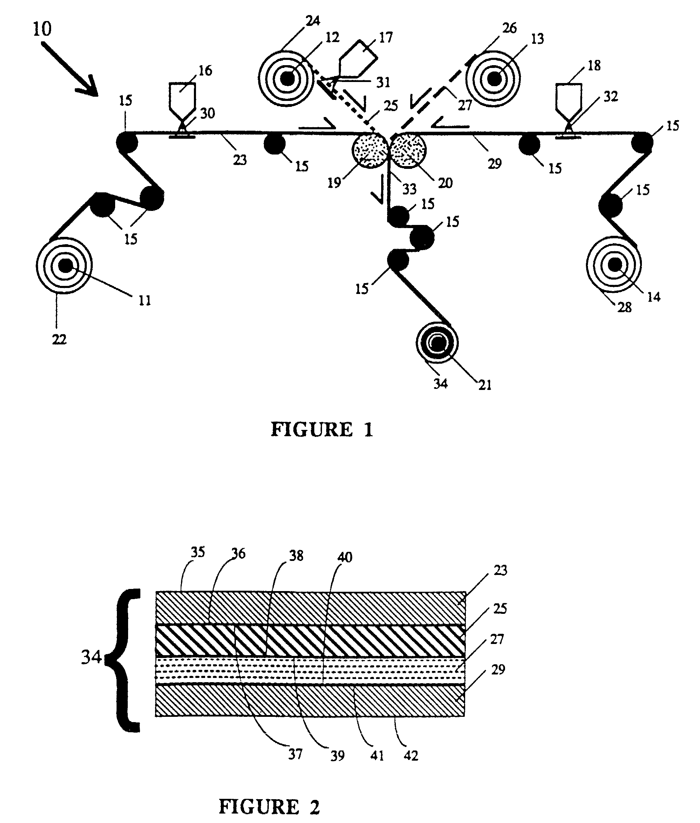

Figure 1 is a schematic illustrating the lamination process for making the composite of the present invention. Figure 2 is an enlarged cross-section of a composite of the present invention.

### DESCRIPTION OF THE PREFERRED EMBODIMENTS

The composites of the present invention exhibit both breathability and resistance to liquids, and are useful in a variety of medical and/or hygiene applications such as protective apparel, wound dressings, sterile dressings, absorbents (e.g. diapers), face masks and the like. Composites used in many of these applications should have the following properties:
(a) breathable,
(b) resistance to liquid,
(c) relatively high strength,
(d) wear resistant,
(e) comfort,
(f) appearance, and
(g) relatively low cost.

In some of the applications, the composite should in addition have absorbency for liquids.

The composite constructed according to the present invention combines the barrier properties of microporous film, the breathability, appearance and strength of nonwovens, and the absorbency and comfort of cotton.

In its broadest embodiment, the composite comprises a core web of staple fibers which exhibits hydrophilic wetting characteristics, and flanking layers of a microporous film and a nonwoven web. Each of the layers of the three-layered structure are adhesively bonded together by a low temperature, low pressure bonding process.

In a preferred embodiment, the composite further includes a second web of a nonwoven adhesively bonded to the outer layer of the microporous film. The description of each of the webs used in the composite, the method of lamination, and properties of the composite, are described below.

### Nonwoven Webs

Nonwoven webs are webs made of randomly oriented fibers or filaments of thermoplastic polymer by entangling the fibers or filaments through mechanical, thermal, or chemical means. Nonwovens exclude paper and products which are woven, knitted, tufted, or felted by wet milling. The preferred nonwoven webs for use in the present invention are the spunbonded and melt-blown webs.

The spunbonded webs are formed by filaments that have been extruded, drawn, laid on a continuous belt, and then immediately thermally bonded by passing through a heated calender. These webs are continuous filament fiber structures having an average fiber diameter between 12 and 50 microns.

Meltblown webs are made by extruding a molten plastic through a row of die openings to form filaments and contacting the extruded filaments with high velocity sheets of converging hot air. The converging air contacts and attenuates or draws the filaments down, depositing them as fibers onto a collector in a random pattern, forming a meltblown web. The meltblown webs have average fiber size between 0.5 to 15 microns which is substantially smaller than the average fiber size of the spunbond web. Another difference between the meltblown and spunbond webs is that the meltblown webs are generally held together by fiber entanglement with some thermal bonding whereas the spunbond webs are generally thermal bonded by the calender, although spunbond webs are also bonded by chemical, adhesive, and needling processes.

Both the spunbonding and meltblowing techniques are well known in the art. For example, U.S. Patent 4,405,297 discloses a spunbond process and U.S. Patent 3,978,185 discloses a meltblowing process, both of which are incorporated herein for reference.

For purposes of the present invention, the nonwoven webs can be made of any synthetic thermoplastic polymer used in meltblowing or spunbond processes. By way of example, these include the following: polyolefins, particularly ethylene and propylene homopolymers and copolymers (including EVA and EMA copolymers), nylon, polyamines, polyester, polystyrene, poly-4-methylpentene, polymethylmethacrylates, polytrifluorochloro-ethylene, polyurethanes, polycarbonates, silicones, polyphenylene sulfide, and polypropylene or polyethylene terephthalate.

The most common polymers used in spunbonded fabrics include polypropylene having a melt flow rate of 12-40. These polymers generally are extruded at temperatures ranging from 180° to 350°C.

The most common polymers used in meltblown fabrics include polypropylene, having melt flow rates of 10-2500.

These polymers are generally extruded at temperatures of between 180 to 350°C and contacted with high velocity air from 180 to 375°C.

The preferred weight of the nonwoven web is from about 0.1 to about 2 oz. per square yard. The preferred weights are between about 0.25 to about 1.5 oz. per square yard for the spunbond webs.

The nonwovens may include additives to impart desired properties to the webs. Examples of these additives are wetting agents, fluorochemicals, antistatics, and anti-microbiotic agents.

As indicated above, the nonwoven webs in a preferred embodiment comprise the outer two layers of the four layer structure. These webs impart strength to the structure, improve the hand (softness to the feel) and wearability, and improve the comfort and appearance giving the composite fabric a clothlike appearance and feel.

### Microporous Film

The term "microporous film" means microporous membrane. The terms film and membrane are used interchangeably herein.

Microporous films are defined as having a narrow pore sized distribution in the submicron range, preferably from 1.0 to 10 microns. The microporous films can be made by a number of processes, which include (a) dissolving polymers in solution followed by extraction of the solvent by water vapor, (b) stretching of crystallizable polymers which results in microsized tears, and (c) stretching of a mineral filled polyolefin film. The polymers used in the microporous films include PTFE, polyolefins, polyurethanes, polyamides, and polyesters.

The preferred microporous film used in the present invention is a polyolefin prepared by stretching a highly filled polyolefin film to impart permeability therein, in accordance with U.S. Patent 4,777,073, the disclosure of which is incorporated herein by reference. The microporous film prepared by this process exhibits excellent breathability, at least 3,000, and generally from 4,000 to 10,000 grams per square meter per day, and in comparison to other microporous film is inexpensive.

Polyolefins used to make the film include polypropylene, copolymers of propylene, homopolymers, and copolymers of ethylene and blends thereof. A preferred polyolefin is a copolymer of polypropylene and low density polyethylene, particularly linear low density polyethylene (LLDPE) . The preferred filler at concentrations of from 30 to 70 wt% include inorganic fillers such as calcium carbonate, TiO2, talc, clay and silica diatomaceous earth, magnesium carbonate, barium carbonate, magnesium sulfate, and the other inorganic fillers listed in the above reference, U.S. Patent 4,777,073.
Calcium carbonate is the preferred filler. The pore size of this film ranges from 0.1 to 0.5 microns.

### Staple Fiber Web

The term "Staple Fibers" as used herein includes natural or synthetic discrete fibers having a length from less than 1 inch to about 8 inches, preferably from about 0.5 inch to about 5 inches and most preferably from about 1 inch to about 3 inches. The staple fibers may include only one type of fibers or may include blends. For purposes of the present invention, the fibers must exhibit at least some hydrophilic properties. The preferred concentration of the hydrophilic fibers in the blend should be at least 25% and preferably more than 50%.

The synthetic man-made fibers may be made from thermoplastics such as polyolefins (including polypropylene and polyethylene), polyesters, and polyamides which are extruded to the proper diameter (usually from 10 to 50 microns) and cut in the desired length, usually from 0.5 to 5.0 inhes. The staple or natural fibers may be any cellulosic base fibers, such as cotton, ramie, hemp, flax, jute, kenaf, bagasse, eucalyptus, rayon, and combinations thereof but do not include wood fiber. The staple fibers may be formed into a web by any of the presently known processes, including, but not limited to, thermal bonding, latex bonding, or carding, or needle-punching, or hydroentangling. The preferred web of staple fibers includes cotton fibers or cotton fibers blended with other staple fibers. The cotton fibers preferably have a fineness of between about 3 to 5 micronaire units to give the web flexibility. The cotton staple fibers have an average width of about 15 to 20 microns.

The staple fibers, which exhibit absorbency for aqueous based materials such as water and blood, act as a reservoir for any liquid or blood. The wicking property of the hydrophilic layer also absorbs water vapor. Moreover, the staple fiber web improves the comfort property of the composite.

### Adhesive

Any of the adhesives compatible with polyolefins and the staple fibers may be used. The preferred adhesives are the hot melt adhesives such as the polypropylene based adhesives, and EVA adhesive, (e.g. 20-40 wt% VA).

### Adhesive Bonding

It is important in laminating the composites of the present invention to use nonthermal bonding techniques. Thermal bonding has a tendency to damage the microporous film by introducing pinholes. The preferred technique for applying the adhesive is by meltspraying or meltblowing of adhesives wherein an air/adhesive spray is deposited on one of the surfaces to be bonded. Both the meltblowing or meltspraying involve extruding a filament or filaments of a hot melt adhesive from a die and contacting the filament or filaments with air to either stretch or attenuate the filament or break it up into droplets which are deposited on the surface of the web. The amount of adhesive deposited on the web may vary within a wide range, but it should be sufficient to ensure good adhesion but not so much that substantial amounts of the pores of the web are plugged. Application from about 1 to 10 grams per square meter of the adhesive should be sufficient, with 1 to 5 grams per square meter being preferred.

As indicated above, the bonding should be by nonthermal pressure techniques. "Nonthermal" means the composite is formed by applying a bonding pressure at temperatures below melting point or softening temperature of the polymers used in the laminate. With polyolefins, this means that the temperatures are carried out below 100°C and preferably below 50°C, most preferably below 30°C. The lower limit of the laminating temperature will be ambient which, depending on the geographic location, may vary widely from 0°C to 50°C.

A laminating apparatus 10 which may be used in laminating the composite of the present invention is schematically illustrated in Figure 1. The laminating assembly comprises a plurality of spindles 11, 12, 13, and 14 for receiving rolls of web and film, guide and tension rollers 15, meltblowing dies 16, 17, and 18, calender rolls 19 and 20, and take-up spindle 21. The various rolls may be mounted on the feed spindles 11-14 in the manner described below. A roll 22 of nonwoven web is mounted on spindle 11, with web 23 dispensed therefrom, trained around rollers 15, disposed under the die 16, and fed into the nip of calender rolls 19 and 20. A roll 24 of microporous film is mounted on spindle 12 with web 25 dispensed therefrom, disposed under die 17, and fed into the nip of calender rolls 19 and 20. A roll 26 of staple fiber is mounted on spindle 13 and web 27 dispensed therefrom and fed into the nip of calender rolls 19 and 20. Finally, a roll 28 of a nonwoven web is mounted on spindle 14 and web 29 dispensed therefrom. Web 29 is trained around rollers 15, passing under meltblowing die 18, and into the nip of calender rolls 19 and 20.

The calender rolls may consist of the following combinations:
(a) driven rolls 19 and 20 having smooth rubber surfaces; or
(b) roll 19 with a smooth steel surface and roller 20 having an embossed pattern in the shape of a raised geometric shape such as diamond or square. The embossment (area of the raised portions contacting the web passing through the nip) ranges from 5 to 35%, preferably from 10 to 25% of the total surface of the web in contact therewith.

In operation, the four webs 23, 25, 27, and 29 are fed in overlaying relation into the nip of the rolls 19 and 20 as shown in Figure 1 and driven or pulled therethrough around guide roll 15 and wound onto spindle 21 forming composite roll 34.

The calender rolls 19 and 20 are maintained at a temperature well below the softening temperature of the polymers, preferably less than 100°C and the pressure at the nip is maintained between about 50 to 150 psi, preferably 75 to 125 psi, using the smooth rolls and about 150 to 250 pounds per linear inch using the combination smooth roll and embossed roll.

As the webs 23, 25, and 29 pass under meltblowing dies 16, 17, and 18, a spray 30, 31, and 32 of adhesives is deposited on a surface of each web. The adhesive deposited on such webs forms a thin discontinuous layer thereon. Deposition of the adhesive in this manner ensures that adhesive will be at the interface of each of the webs as they are passed through the nip of the calender rolls 19 and 20. The speed at which the webs are processed through the nips may vary within relatively wide ranges, but speeds of 5 to 10 meters per minute are sufficient to ensure good bonding.

As illustrated in Figure 2, the composite 34 comprises a first nonwoven web 23 having outer surface 35 and an inner surface 36 adhesively bonded to surface 37 of microporous film 25. The opposite surface 38 of microporous film 25 is adhesively bonded to surface 39 of staple fiber web 27. Likewise, surface 40 of staple fiber web 27 is adhesively bonded to surface 41 of the second nonwoven web 29. Surface 42 of web 29 when used as protective apparel will be in contact with the wearer's body, and outer surface 35 of the composite 34 will be exposed to the environment. Thus, any blood or toxic liquid of the environment coming into contact with the apparel will first have to pass through the nonwoven web 23 and then through the barrier microporous film 25. The microporosity and the hydrophobic nature of these layers will act as a barrier for the liquid. Any liquid that does pass these barrier layers will enter the hydrophilic staple fiber layer which will act as a reservoir or wicking layer for containing the blood or the liquid. Finally, the inner nonwoven layer 29 acts as a second barrier, though not nearly as effective as the microporous film 25.

A variation of the lamination apparatus 10 used in the process includes basically the same apparatus without spindle 12 and die 17. In this assembly, one pass is required for three-layer laminates and two passes are required for four-layer laminates.

The breathability of the composite 34 permits air and water vapor to pass sequentially through layers 29, 27, 25, 23, and into the environment, thus providing comfort for the wearer. The staple fiber layer 27 provides for the additional function of acting as a wicking material for any perspiration or liquid that might pass from the wearer outwardly.

The following table presents some of the preferred properties or specifications for each layer.

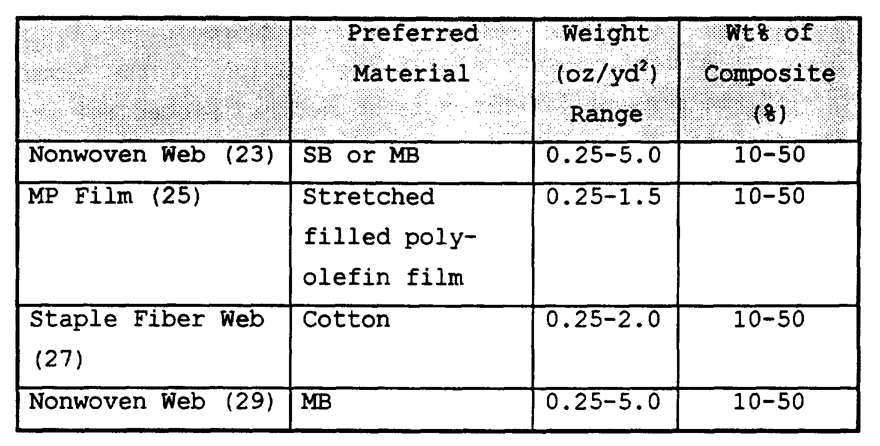

The composite 34 preferably has a thickness ranging from 0.2 to 1.5 mils, most preferably 0.3 to 1.0 mils, and an MVTR of at least 400 g/m²/24 hrs. and most preferably at least 500 g/m²/24 hrs. MVTR of 500-1000 g/m²/24 hrs. will be satisfactory for most composites. The composite has a bursting strength of at least 10 psi and preferably of at least 15 psi. The composite may be made in weight ranging from 2.00 to 6.00 oz/yd², preferably 2.5 to 4.0 oz/yd².

### Uses of Composites

As amply demonstrated by the test results presented below, the composite of the present invention exhibits a combination of properties making it ideally suited for a number of applications:
(a) The composite is breathable (see MVTR Test results).
(b) The composite exhibits good barrier to liquids (see Blood and Viral Resistance Test results).
(c) The composite has a soft, clothlike hand and appearance.
(d) The composite with the preferred microporous film (EXXAIRE^{B}) costs less than many composites having other microporous films.

A particularly useful application of the composite of the present invention is in medical protective apparel designed to protect the wearer from contact with external blood or toxic liquids, or to protect the environment from blood or liquid contamination emanating from the wearer.

Other medical and hygiene uses include feminine hygiene absorbents, baby diapers, adult incontinents, industrial protective apparel, wound dressing, transdermal patches, and the like. Other uses include sportswear, rain gear, footwear, and the like.

Although the reasons for the improved performance (MVTR and Blood and Virus Resistance Tests) of the composite of the present invention are not fully understood, it is believed that the combination of the microporous film and the nonwoven layers improve barrier properties and the staple fibers provide a hydrophilic reservoir or wicking layer for the blood or aqueous liquids.

### EXPERIMENTS

Experiments were carried out to test the properties of laminates made in accordance with the present invention.
Web Materials: The three layer and four layer laminates were made of the following materials:
- MB -: a polypropylene meltblown nonwoven web having an average fiber diameter of between 3.8 to 4.3 microns;
- SB -: a polypropylene spunbond web having an average fiber diameter of between 23.0 microns;
- MP -: a polyethylene microporous film marketed by EXXON Chemicals Company as "EXXAIRE";
- Cotton -: staple fiber cotton which has been carded (C), or thermally bonded (TC), or latex bonded (LC);
- Adh. -: a PP based hot melt adhesive marketed by Finley Adhesive, Wauwatosa, Wisconsin, as H2279.
Laminate Process: Laminate structures were made as follows:
a) Three layer composites were made by feeding into the nip of counterrotating calender rolls from three separate webs in overlaid relation. Prior to entering the nip the two outer webs were passed under a meltblowing die which meltblew a hot melt adhesive thereon at about 5.0 g/m². The surface of each outer web thus was bonded to the middle web of the three layer structure and the three layer composite was wound into a roll.
(b) The four layer composite was made by preparing the three layer composite as described above followed by the step of feeding into the nip of calender rolls the three layer composite as described above and a fourth web from a fourth web roll. Prior to entering the nip, the 3-layer composite and the fourth web were each passed under a meltblowing die where an adhesive (H2279) was applied at about 5.0 g/m². The surfaces with the adhesive were fed into the nip to bond with each other. The four layer structure was bonded together and wound on a roll.
Smooth calender rolls (SC) or embossed calender rolls (EC) were used to bond the different layers.

The smooth calender consisted of two rolls provided with smooth rubber covers. The embossed calender consisted of a raised diamond pattern steel roll and a smooth steel roll. The raised area of the diamond pattern represented about 14.7% roll area.
In calendering with the smooth rolls, the calender rolls were operated at a nip temperature of about 20°C, a nip pressure of about 80-90 psi and a speed of about 5-10 m/min. The point bonding with the embossed rolls (Kusters Two Roll) was carried out at a nip temperature of 23°C, a nip pressure of 150 or 200 PLI, and a nip speed of 5-10 m/min.
The smooth calendered composites (having four layers) were prepared by two passes through the calender nip. In the first pass, three layers were laminated with only minimal nip pressure. In the second pass, a fourth web was added and then passed through the calender nip at the conditions described above.
In point bonding, the four layers were combined by the two-pass method described above except the nip pressure in both passes was minimal. These laminates were then bonded by passing through the nip of the embossed calender at conditions described above.
Laminate Structures: Duplicate multilayered structures were made; the webs of one being adhesively bonded together by the smooth calender (SC) and the other by the embossed calender (EC) as described above.

### SERIES I TESTS:

The SERIES I TESTS were carried out on composites comprising the following layers:
(a) a web of MB having a weight of 0.50 (Samples 1, 3, 5, 7) or 0.75 (Samples 2, 4, 6, and 8) oz/yd²;
(b) an MP film having a weight of 0.94 oz/yd² except for Samples 3 and 4 which was 0.50 oz/yd²;
(c) a web of cotton (C or TC or LC) having a weight of 0.60 (Samples 7 and 8), 0.65 (Samples 5 and 6), or 0.75 (Samples 1 thru 4);
(d) a web of MB having a weight of 0.50 or 0.75 [same sample distribution as web (a)].

The laminates were adhesively bonded by meltblowing an adhesive onto a surface of layers (a), (b), and (c).

The SERIES I TEST composites had the following specifications:

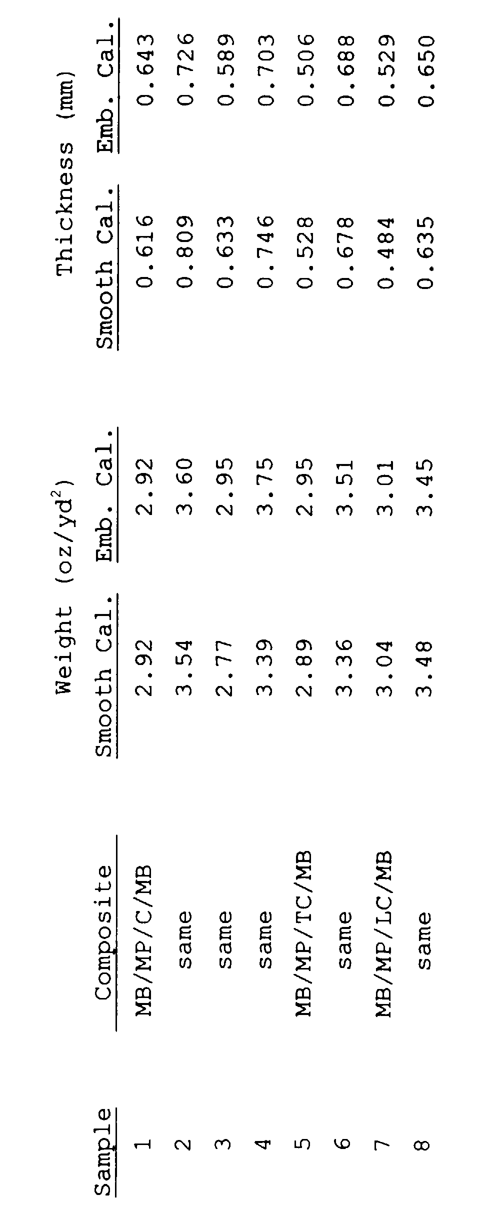

### SERIES II TESTS:

These tests were carried out on four-layer composites as described for Sample 1 in the SERIES I TEST Samples except a polypropylene spunbond web having a weight of 0.60 oz/yd was used as layer (a). The SERIES II composites had the following specifications:

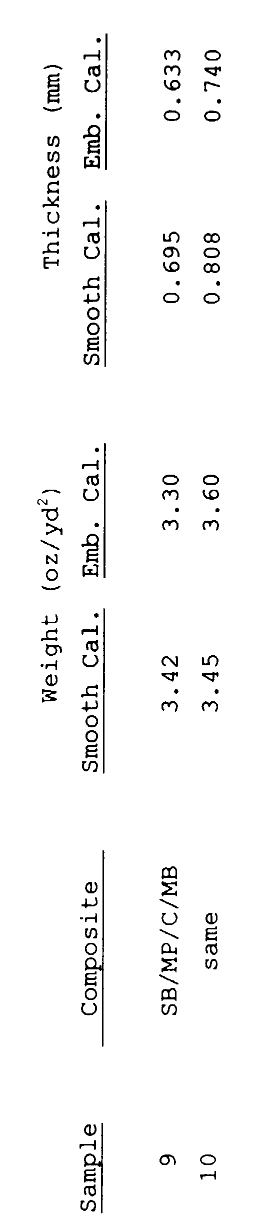

### SERIES III TESTS:

These tests were carried out using four layer composites as described in the SERIES I TESTS except layers (a) and (d) were polypropylene spunbond webs having a weight of 0.60 oz/yd². The MP film had a weight of 0.50 for all samples except for Sample 11 (0.94 oz/yd²). The cotton layer for the samples had weights of 0.75 (Samples 11 and 12), 0.65 (Sample 13), or 0.60 (Sample 14). SERIES III TEST composites had the following specifications:

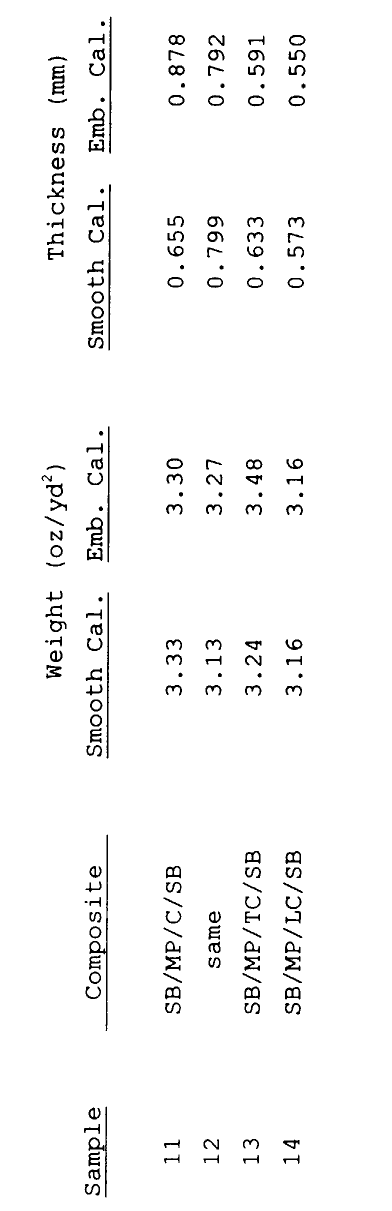

### COMPARATIVE SAMPLES:

Six laminates without the cotton web and three laminates without the MP film were made and tested. Layers (a), (b), and (d) of Samples 15, 16, 17, 18, 19, and 20 corresponded to the same layers of Samples 1, 3, 2, 4, 9, and 11, respectively. Layers (a), (c), and (d) of Samples 21, 22, and 23 corresponded to the same layers of Samples 1, 2, and 3, respectively. These comparative samples had the following specifications:

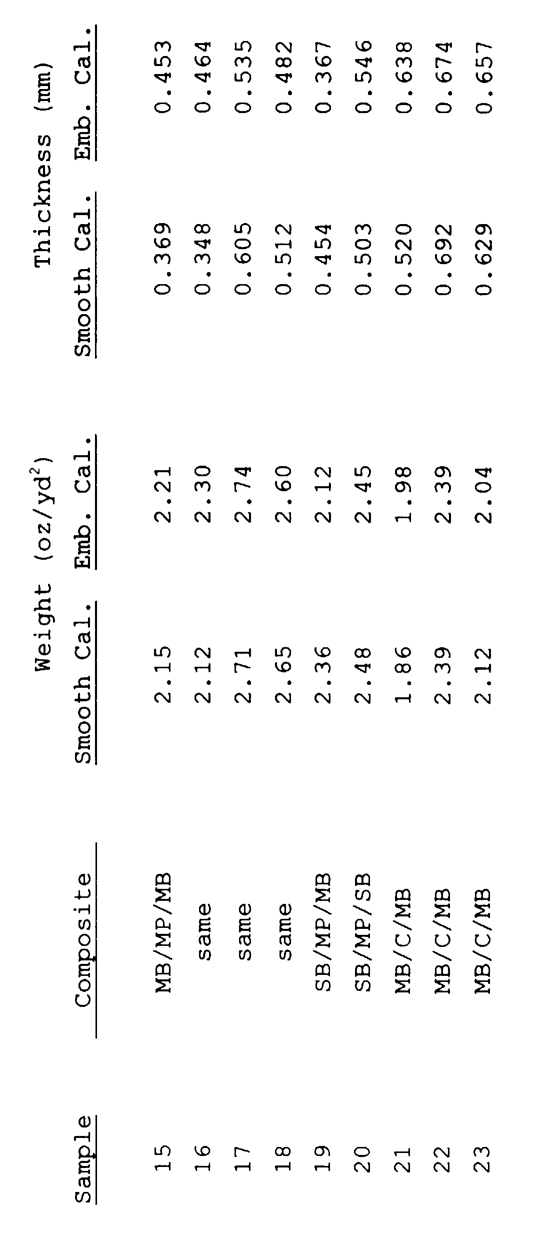

### TEST PROCEDURES:

The following tests were performed on each sample:

| | |
|---|---|
| Bursting Strength | INDA Standard Test (INDA, Association of the Nonwoven Fabrics Industry) 1st 30.0 - 70 (R82) |
| | |
| MVTR (Moisture Vapor Transmsission Rate) | ASTM E96-80 |
| | |
| Resistance of Protective Clothing Materials to Synthetic Blood | (ASTM Designation: ES 21-92): This test method covers the determination of the resistance of protective clothing materials to penetration by biological liquids using synthetic blood under the condition of continuous liquid contact. Protective clothing material "pass/fail" determinations are based on visual detection of synthetic blood penetration. |
| | |
| Resistance of Protective Clothing Materials to Penetration by Blood-Borne Pathogens Using Viral Penetration as a Test System | (ASTM Designation: ES 22-92) This test method is used to measure the resistance of protective clothing materials to penetration to blood-borne pathogens by using a surrogate microbe under the condition of con-tinuous liquid contact. Protective clothing "pass/fail" determinations are based on detection of viral penetration. |

TEST RESULTS: The following Table presents the results of the tests carried out:

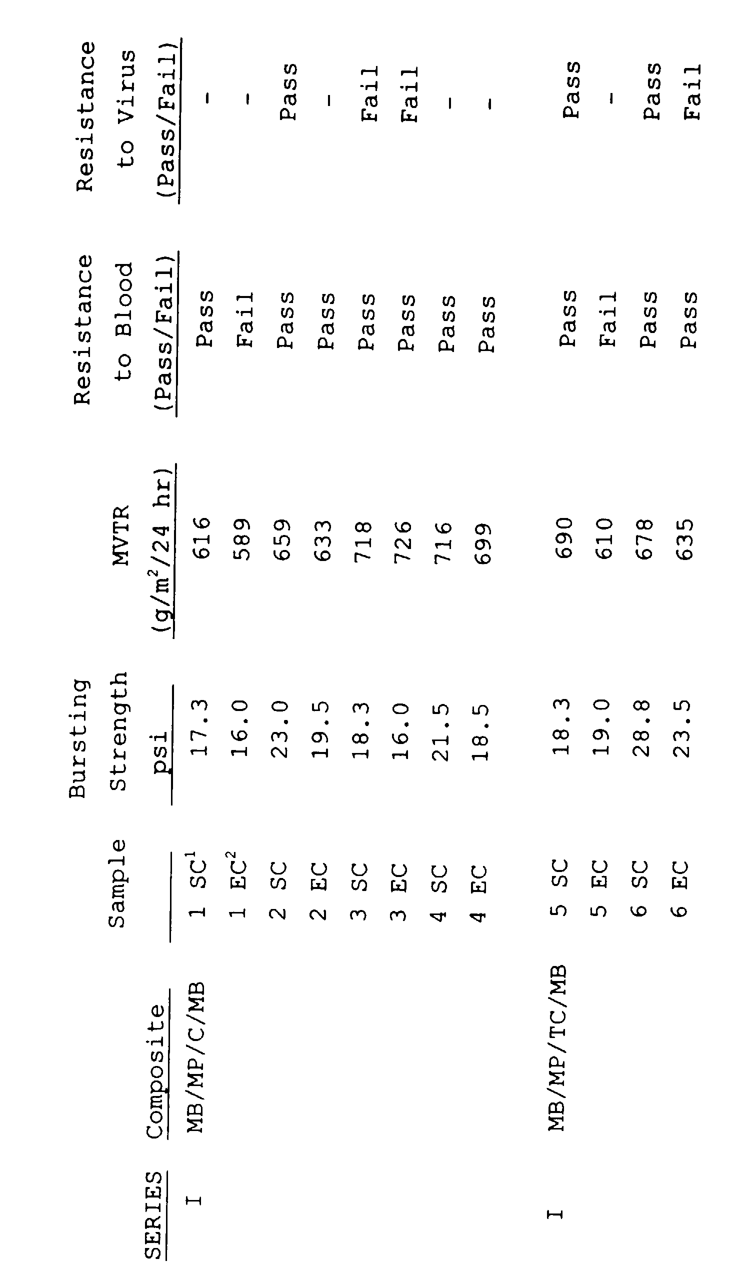

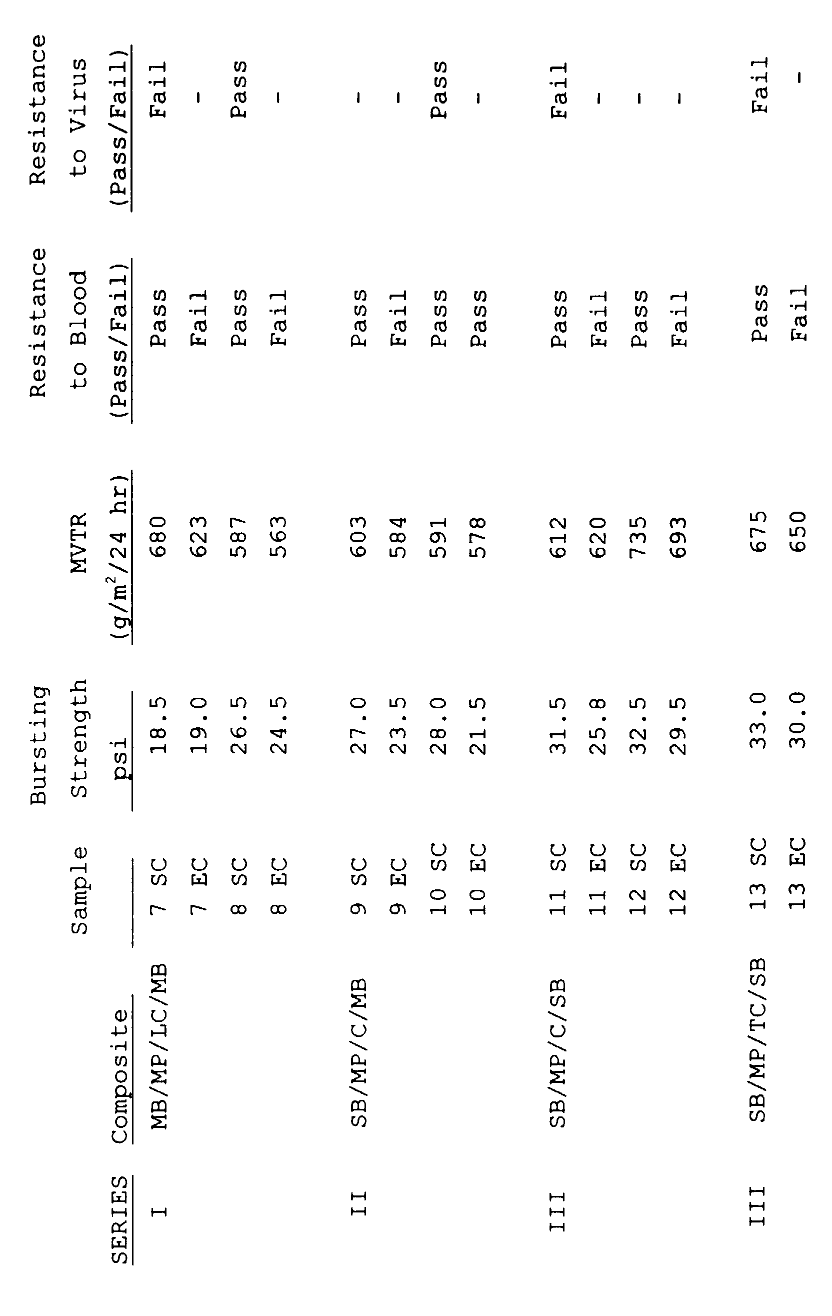

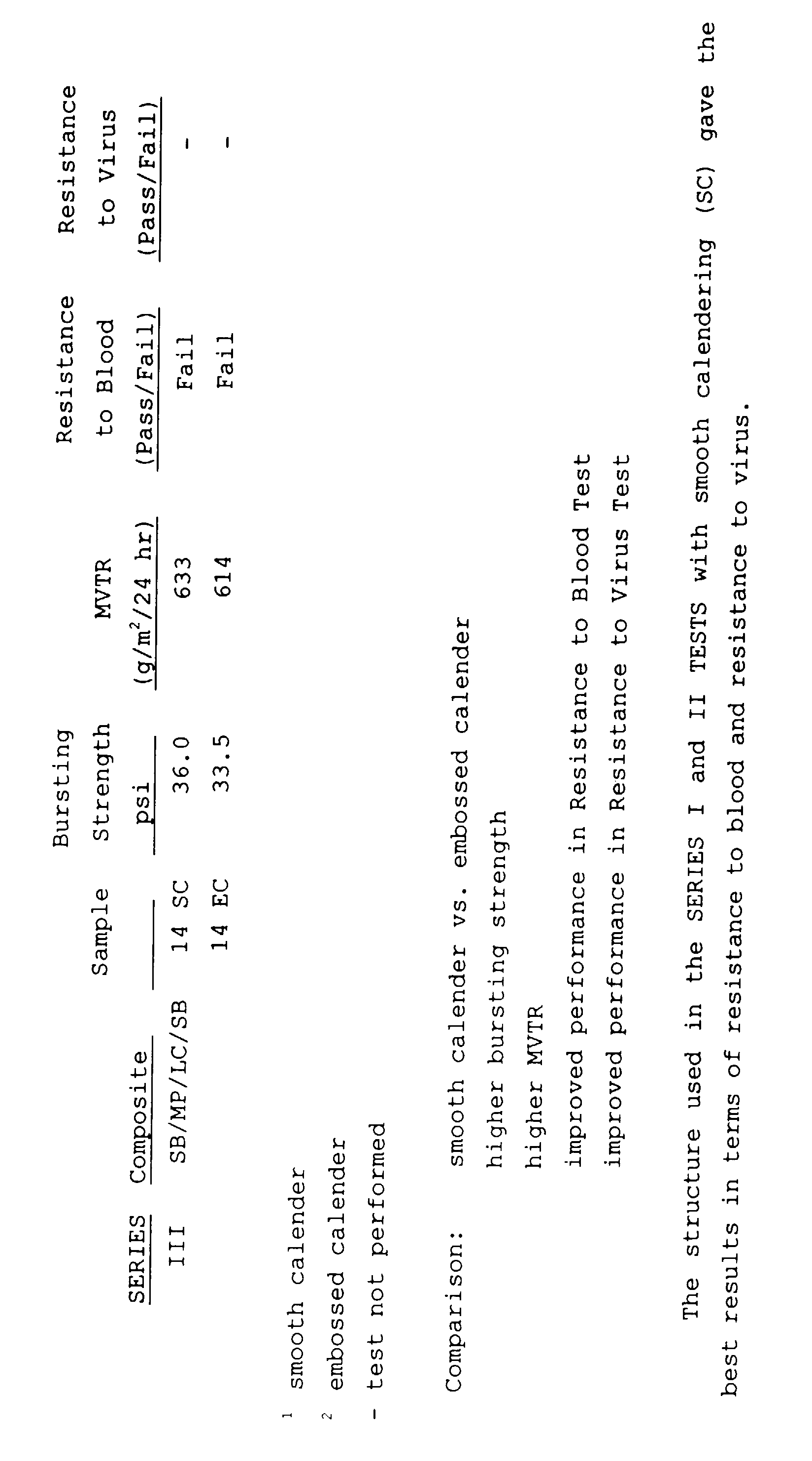

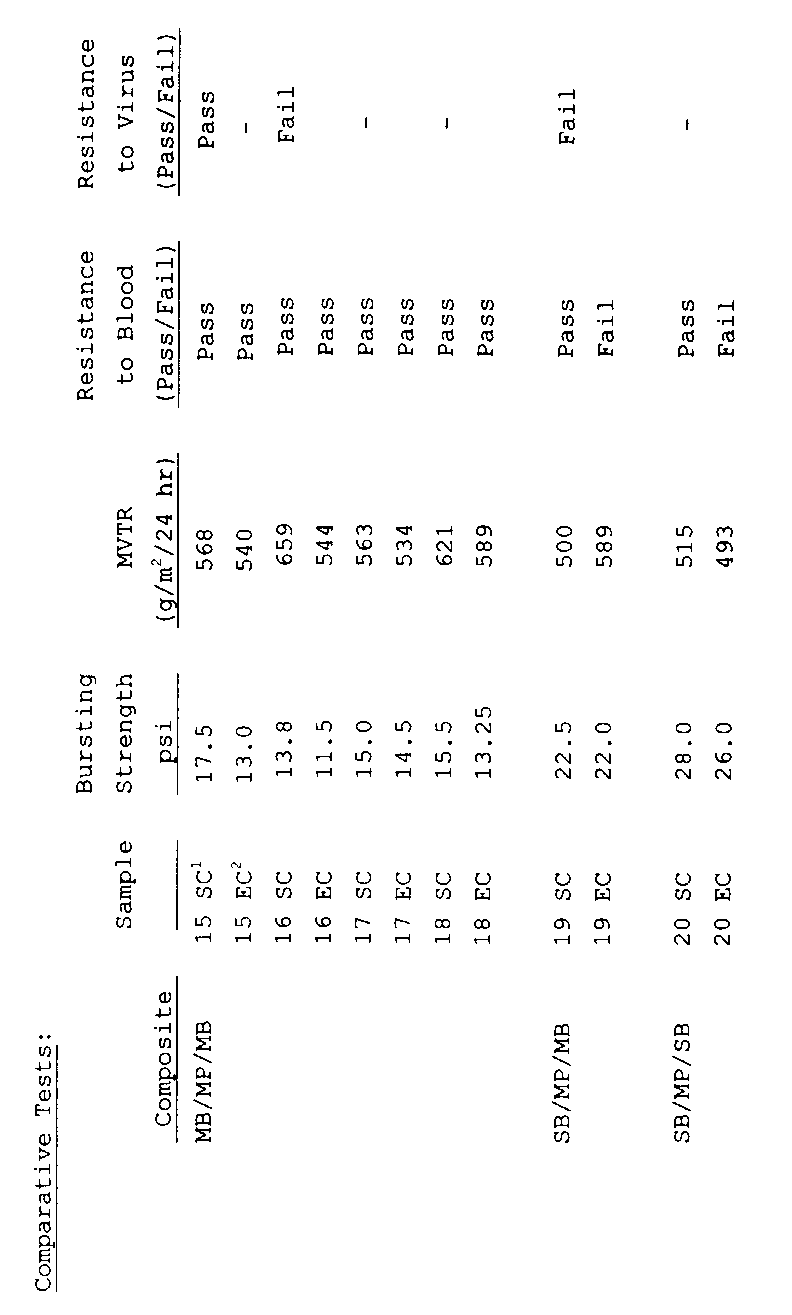

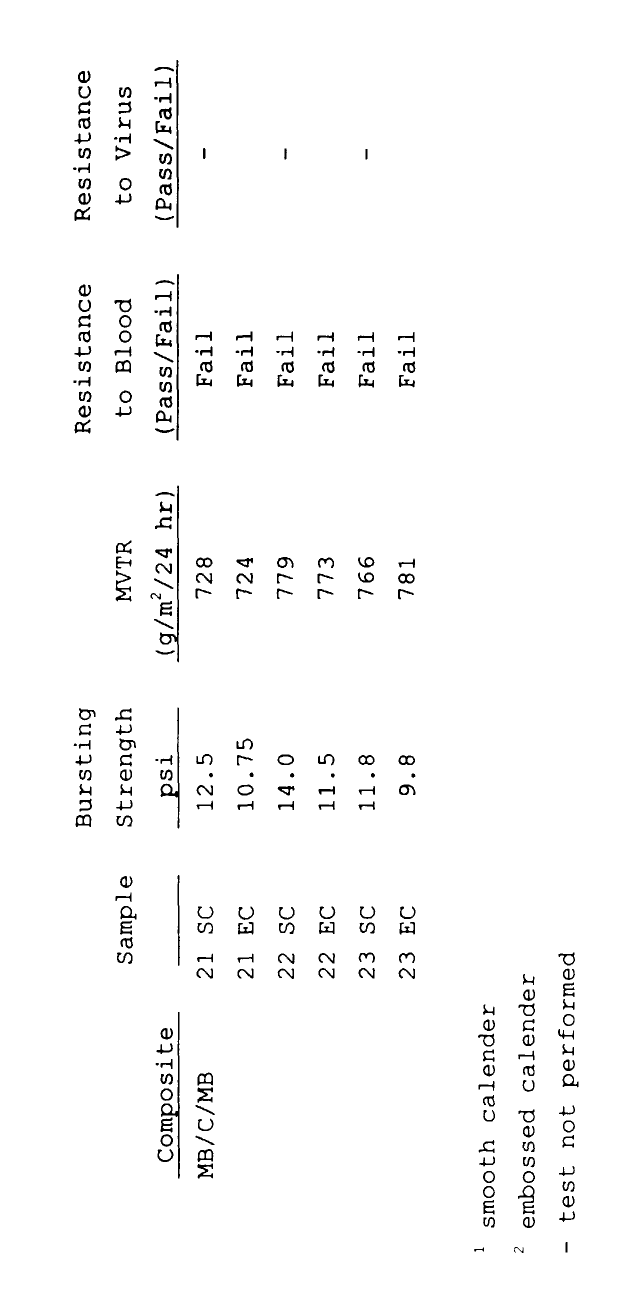

It is significant to note that the comparative composites (Samples 15, 16, 17, 18, 19, and 20) vis-a-vis its corresponding composites of the present invention (Samples 1, 3, 2, 4, 9, 11) with cotton were (a) consistently lower in bursting strength and (b) consistently lower (except for Sample 19 EC) in MVTR.

All of the Comparative Samples (except Samples 19EC and 20EC) without the cotton passed the Resistance to Blood Test but only one of the three tested passed the more rigorous Resistance to Virus Test.

The data clearly demonstrates the effect of staple fibers (e.g. cotton) on the composites' bursting strength and MVTR and suggests that it plays an important role in the Resistance to Blood and Virus.

It required only the testing of three of the Comparative Samples (21, 22, and 23) to demonstrate the importance of the MP film. None of these samples passed the Resistance to Blood Test, and all had low bursting strengths.

Although the composites of the present invention have been described as comprising three or four layers adhesively bonded together, it is to be emphasized that this represents the preferred structure. Variations include adhesively bond-ing intermediate layers between two or more of the recited layers.

## Claims

1. A laminate comprising:
(a) a first inner layer of a microporous film;
(b) a second inner layer overlaying the first inner layer and being composed of staple fibers, at least 25 vol% of which are hydrophilic staple fibers; and
(c) first and second outer layers of thermoplastic non-woven webs positioned on opposite sides of the overlaid inner layers, said non-woven webs having an average fiber or filament size of 0.5 to 30 microns,
said layers being bonded together by adhesives placed at the layer interfaces.

2. A laminate as claimed in claim 1, wherein the second inner layer comprises at least 25 wt% of cotton fibers.

3. A laminate as claimed in claim 2, wherein the cotton fibers are selected from the group consisting of carded fibers, latex bonded fibers and thermally bonded fibers.

4. A laminate as claimed in claim 2 or 3, wherein the second inner layer comprises at least 50 wt% of cotton fibers.

5. A laminate as claimed in claim 4, wherein the second inner layer comprises 100% of cotton fibers.

6. A laminate as claimed in any one of the preceding claims, wherein the second non-woven layer is a meltblown layer having an average fiber size of 0.5 to 15 microns.

7. A laminate as claimed in any one of the preceding claims, wherein both of the outer non-woven layers are composed of meltblown webs having an average fiber diameter of from 0.5 to 15 microns.

8. A laminate as claimed in any one of the preceding claims, wherein the microporous film has an average pore size of 1.0 to 10 microns.

9. A laminate as claimed in any one of the preceding claims, wherein the laminate has a thickness of from 0.2 to 1.5 mils and has the following properties: moisture vapor transmission rate of at least 400 g/m² per 24 hrs, and a bursting strength of at least 15 psi.

10. A laminate as claimed in any one of the preceding claims, wherein the laminate is capable of passing the Resistance to Blood Penetration Test of ASTM ES 21-92.

11. A laminate as claimed in any one of the preceding claims, wherein the laminate is capable of passing the Resistance to Virus Test of ASTM ES 22-92.

12. A laminate as claimed in any one of the preceding claims, wherein the microporous film is a particle filled polyolefin stretched to provide permeability therein.

13. A laminate as claimed in any one of the preceding claims, wherein the microporous film represents at least 25 wt% of the laminate.

14. A laminate as claimed in any one of the preceding claims, wherein the adhesive is hot melt adhesive.

15. A laminate as claimed in claim 14, wherein the adhesive bonding the layers together is from 1 to 10 grams/m².

16. A laminate as claimed in claim 14 or 15, wherein the adhesive is in the form of layers which have been sprayed onto the laminate surfaces and thereafter pressure bonded at a temperature less than 100°C.

17. A laminate suitable for medical uses which comprises:
(a) a first non-woven web selected from the group consisting of thermoplastic spunbond and meltblown webs and having an outer surface and an inner surface;
(b) a microporous film having an outer surface facing the first non-woven web and adhesively bonded thereto, and an inwardly facing surface;
(c) a staple fiber web comprising at least 25 wt% of hydrophilic fibers, and having an outer surface adhesively bonded to the inwardly facing surface of the microporous film, and an inwardly facing surface;
(d) a second non-woven web selected from the group consisting of thermoplastic meltblown web of spunbond and meltblown webs, and having an outer surface adhesively bonded to the inwardly facing surface of the staple fiber web.

18. A laminate as claimed in claim 17, wherein the microporous film is composed of films selected from the group consisting of polyolefins, polyamides, polyurethanes, and PTFE.

19. A laminate as claimed in claim 17 or 18, wherein the staple fiber web comprises more than 50 wt% of cotton fibers.

20. A laminate as claimed in any one of claims 17 to 19, wherein the microporous film is prepared by stretching a polyolefin having inorganic filler dispersed thereon to impart sufficient porosity thereto to provide the film with breathability.

21. A method of making a laminate comprising:
(a) selecting a layer of a first non-woven web and a layer of microporous film;
(b) melt spraying an adhesive to a surface of either the non-woven web or the microporous film;
(c) overlaying the first non-woven web and the microporous film with the sprayed on adhesive disposed therebetween, the microporous film having an inner surface facing the first non-woven web and an outer surface;
(d) selecting a layer of a second non-woven web and a staple fiber layer;
(e) melt spraying an adhesive to either the surface of the second non-woven web or the staple fiber layer;
(f) overlaying the second non-woven web and the staple fiber layer with the sprayed on adhesive disposed therebetween, said staple fiber layer having an inner surface facing the second non-woven web and an outer surface;
(g) spraying an adhesive on either the outward surface of the microporous film or the second non-woven web;
(h) feeding the four layers in overlaid relation through the nip of pressurized counterrotating calender rolls wherein adhesive is at the interface of each of the layers, whereby the layers are pressure bonded together by the adhesives.

22. A method as claimed in claim 21, wherein the staple fibers comprise more than 50 wt% of cotton fibers.

23. A method as claimed in claim 21 or 22, wherein the first non-woven web is selected from the group consisting of meltblown and spunbond webs and the second non-woven is a meltblown web.

24. A method as claimed in any one of claims 21 to 23, wherein the pressure is from 10 to 200 psi.

25. A method as claimed in any one of claims 21 to 24, wherein the surface of the nip rolls is made of smooth resilient material.

26. A method as claimed in any one of claims 21 to 25, wherein the surface of both rolls is smooth.

27. A method as claimed in any one of claims 21 to 26, wherein one of the calender roll surfaces is embossed.

28. A method as claimed in any one of claims 21 to 27, wherein the adhesive is a hot melt adhesive.

29. A method as claimed in any one of claims 21 to 28, wherein the temperature of the rolls is maintained at less than 100°C.

30. A protective apparel for covering a body member to protect the body member from contact with blood or extraneous liquids which comprises
(a) a first thermoplastic non-woven layer having an outer surface being disposed outwardly from the body member, and an inner surface facing the body member;
(b) a staple fiber layer having an outer surface facing outwardly from the body member, and an inner surface facing the first non-woven layer and being adhesively bonded to the outer surface of the first non-woven layer;
(c) a microporous film member having an outer surface facing outwardly from the body member and an inner surface facing the outer surface of the staple fiber layer and being adhesively bonded thereto;
(d) a second thermoplastic non-woven layer having an outer surface facing outwardly from the body member and an inner surface adhesively bonded to the outer surface of the microporous film layer.

31. An apparel as claimed in claim 30, wherein the staple fiber layer includes staple cotton fibers and the second non-woven layer comprises a meltblown web.

32. A laminate comprising a core layer of staple cotton fibers and flanking layers of a microporous film and a non-woven layer selected from the group consisting of thermoplastic meltblown or spunbond fibers or filaments, said flanking layers being adhesively bonded to opposite surfaces of the core layer.

## Patentansprüche

1. Laminat, umfassend:
(a) eine erste innere Schicht aus einem mikroporösen Film;
(b) eine zweite innere Schicht, die die erste innere Schicht überlagert und aus Stapelfasern besteht, von denen wenigstens 25 Vol.-% hydrophile Stapelfasern sind; und
(c) erste und zweite äußere Schichten aus thermoplastischen Vliesbahnen, die auf gegenüberliegenden Seiten der darüberliegenden inneren Schichten angeordnet sind, wobei die genannten Vliesbahnen eine durchschnittliche Faser- oder Filamentgröße von 0,5 bis 30 Mikron aufweisen,
wobei die genannten Schichten durch an den Schichtberührungsflächen vorgesehene Klebstoffe miteinander verbunden sind.

2. Laminat wie in Anspruch 1 beansprucht, bei dem die zweite innere Schicht wenigstens 25 Gew.-% Baumwollfasern aufweist.

3. Laminat wie in Anspruch 2 beansprucht, bei dem die Baumwollfasern aus der Gruppe ausgewählt sind, die aus kardierten Fasern, latexgebundenen Fasern und wärmegebundenen Fasern besteht.

4. Laminat wie in Anspruch 2 oder 3 beansprucht, bei dem die zweite innere Schicht wenigstens 50 Gew.-% Baumwollfasern umfaßt.

5. Laminat wie in Anspruch 4 beansprucht, bei dem die zweite innere Schicht 100% Baumwollfasern umfaßt.

6. Laminat wie in einem der vorhergehenden Ansprüche beansprucht, bei dem die zweite Vliesschicht eine Schmelzblasschicht mit einer durchschnittlichen Fasergröße von 0,5 bis 15 Mikron darstellt.

7. Laminat wie in einem der vorhergehenden Ansprüche beansprucht, bei dem beide der äußeren Vliesschichten aus Schmelzblasbahnen mit einem durchschnittlichen Faserdurchmesser von 0,5 bis 15 Mikron bestehen.

8. Laminat wie in einem der vorhergehenden Ansprüche beansprucht, bei dem der mikroporöse Film eine durchschnittliche Porengröße von 1,0 bis 10 Mikron aufweist.

9. Laminat wie in einem der vorhergehenden Ansprüche beansprucht, bei dem das Laminat eine Dicke von 0,2 bis 1,5 Mil und die folgenden Eigenschaften aufweist: Wasserdampfdurchlässigkeit von wenigstens 400 g/m² pro 24 Std., und eine Bruchfestigkeit von wenigstens 15 psi (pound per square inch - Pfund pro Quadratzoll).

10. Laminat wie in einem der vorhergehenden Ansprüche beansprucht, bei dem das Laminat in der Lage ist, den Blutdichtheitstest von ASTM ES 21-92 zu bestehen.

11. Laminat wie in einem der vorhergehenden Ansprüche beansprucht, bei dem das Laminat in der Lage ist, den Virusresistenztest von ASTM ES 22-92 zu bestehen.

12. Laminat wie in einem der vorhergehenden Ansprüche beansprucht, bei dem der mikroporöse Film ein mit Partikeln gefülltes Polyolefin ist, das zum Schaffen von Durchlässigkeit in demselben gedehnt ist.

13. Laminat wie in einem der vorhergehenden Ansprüche beansprucht, bei dem der mikroporöse Film wenigstens 25 Gew.-% des Laminats darstellt.

14. Laminat wie in einem der vorhergehenden Ansprüche beansprucht, bei dem der Klebstoff Heißkleber darstellt.

15. Laminat wie in Anspruch 14 beansprucht, bei dem der die Schichten zusammenbindende Klebstoff von 1 bis 10 g/m² beträgt.

16. Laminat wie in Anspruch 14 oder 15 beansprucht, bei dem der Klebstoff in Form von Schichten vorliegt, die auf die Laminatoberflächen gesprüht wurden und danach bei einer Temperatur von weniger als 100°C durch Druck gebunden werden.

17. Für medizinische Verwendungen geeignetes Laminat, das umfaßt:
(a) eine erste Vliesbahn, die aus der aus thermoplastischen Spinn- und Schmelzblasbahnen bestehenden Gruppe ausgewählt ist und eine äußere Oberfläche und eine innere Oberfläche aufweist;
(b) einen mikroporösen Film mit einer äußeren Oberfläche, die zu der ersten Vliesbahn gerichtet ist und klebend an dieselbe gebunden ist, und eine nach innen gerichtete Oberfläche;
(c) eine Stapelfaserbahn, die wenigstens 25 Gew.-% hydrophile Fasern umfaßt und eine äußere Oberfläche, die klebend an die nach innen gerichtete Oberfläche des mikroporösen Films gebunden ist, und eine nach innen gerichtete Oberfläche aufweist;
(d) eine zweite Vliesbahn, die aus der aus thermoplastischer Schmelzblasbahn von Spinn- und Schmelzblasbahnen bestehenden Gruppe ausgewählt ist, und eine äußere Oberfläche aufweist, die klebend an die nach innen gerichtete Oberfläche der Stapelfaserbahn gebunden ist.

18. Laminat wie in Anspruch 17 beansprucht, bei dem der mikroporöse Film aus Filmen besteht, die aus der aus Polyolefinen, Polyamiden, Polyurethanen und PTFE bestehenden Gruppe ausgewählt sind.

19. Laminat wie in Anspurch 17 oder 18 beansprucht, bei dem die Stapelfaserbahn mehr als 50 Gew.-% Baumwollfasern umfaßt.

20. Laminat wie in einem der Ansprüche 17 bis 19 beansprucht, bei dem der mikroporöse Film durch Dehnen eines Polyolefins mit einem darauf verstreuten anorganischen Füllstoff hergestellt wird, um demselben ausreichende Porosität zum Versehen des Films mit Atmungsvermögen zu verleihen.

21. Verfahren zum Herstellen eines Laminats, umfassend:
(a) Auswählen einer Schicht von einer ersten Vliesbahn und einer Schicht eines mikroporösen Films;
(b) Schmelzsprühen eines Klebstoffs auf eine Oberfläche entweder der Vliesbahn oder des mikroporösen Films;
(c) Überlagern der ersten Vliesbahn und des mikroporösen Films mit dem dazwischen angeordneten aufgesprühten Klebstoff, wobei der mikroporöse Film eine zu der ersten Vliesbahn gerichtete innere Oberfläche und eine äußere Oberfläche aufweist;
(d) Auswählen einer Schicht von einer zweiten Vliesbahn und einer Stapelfasersch icht;
(e) Schmelzsprühen eines Klebstoffs entweder auf die Oberfläche der zweiten Vliesbahn oder der Stapelfaserschicht;
(f) Überlagern der zweiten Vliesbahn und der Stapelfaserschicht mit dem dazwischen angeordneten aufgesprühten Klebstoff, wobei die genannte Stapelfaserschicht eine zu der zweiten Vliesbahn gerichtete innere Oberfläche und eine äußere Oberfläche aufweist;
(g) Sprühen eines Klebstoffs entweder auf die äußere Oberfläche des mikroporösen Films oder der zweiten Vliesbahn;
(h) Führen der vier Schichten in übereinanderliegendem Verhältnis durch den Walzenspalt von unter Druck stehenden, gegenläufigen Kalanderwalzen, wobei sich Klebstoff an der Berührungsfläche jeder der Schichten befindet, wodurch die Schichten durch die Klebstoffe mit Druck aneinander gebunden werden.

22. Verfahren wie in Anspruch 21 beansprucht, bei dem die Stapelfasern mehr als 50 Gew.-% Baumwollfasern umfassen.

23. Verfahren wie in Anspruch 21 oder 22 beansprucht, bei dem die erste Vliesbahn aus der aus Schmelzblas- und Spinnbahnen bestehenden Gruppe ausgewählt ist und das zweite Vlies eine Schmelzblasbahn ist.

24. Verfahren wie in einem der Ansprüche 21 bis 23 beansprucht, bei dem der Druck von 10 bis 200 psi beträgt.

25. Verfahren wie in einem der Ansprüche 21 bis 24 beansprucht, bei dem die Oberfläche der Abquetschwalzen aus glattem elastischem Material hergestellt ist.

26. Verfahren wie in einem der Ansprüche 21 bis 25 beansprucht, bei dem die Oberfläche beider Walzen glatt ist.

27. Verfahren wie in einem der Ansprüche 21 bis 26 beansprucht, bei dem eine der Kalanderwalzenoberflächen geprägt ist.

28. Verfahren wie in einem der Ansprüche 21 bis 27 beansprucht, bei dem der Klebstoff ein Heißkleber ist.

29. Verfahren wie in einem der Ansprüche 21 bis 28 beansprucht, bei dem die Temperatur der Walzen bei weniger als 100°C gehalten wird.

30. Schutzkleidung zum Bedecken eines Körperteils zum Schützen des Körperteils gegen Kontakt mit Blut oder Fremdflüssigkeiten, die umfaßt:
(a) eine erste thermoplastische Vliesschicht mit einer nach außen von dem Körperteil angeordneten äußeren Oberfläche, und einer zu dem Körperteil gerichteten inneren Oberfläche;
(b) eine Stapelfaserschicht mit einer von dem Körpterteil nach außen gerichteten äußeren Oberfläche und einer inneren Oberfläche, die zu der ersten Vliesschicht gerichtet und klebend an die äußere Oberfläche der ersten Vliesschicht gebunden ist;
(c) ein mikroporöses Filmelement mit einer von dem Körperteil nach außen gerichteten äußeren Oberfläche und einer inneren Oberfläche, die zu der äußeren Oberfläche der Stapelfaserschicht gerichtet und klebend an dieselbe gebunden ist;
(d) eine zweite thermoplastische Vliesschicht mit einer von dem Körperteil nach außen gerichteten äußeren Oberfläche und einer inneren Oberfläche, die klebend an die äußere Oberfläche der mikroporösen Filmschicht gebunden ist.

31. Kleidung wie in Anspruch 30 beansprucht, bei der die Stapelfaserschicht Stapelbaumwollfasern einschließt und die zweite Vliesschicht eine Schmelzblasbahn umfaßt.

32. Laminat, das eine Kernschicht aus Stapelbaumwollfasern und flankierende Schichten eines mikroporösen Films und einer Vliesschicht ausgewählt aus der Gruppe umfaßt, die aus thermoplastischen Schmelzblas- oder Spinnfasern oder
-filamenten besteht, wobei die genannten flankierenden Schichten klebend an gegenüberliegende Oberflächen der Kernschicht gebunden sind.

## Revendications

1. Stratifié comprenant:
(a) une première couche interne d'un film microporeux;
(b) une deuxième couche interne recouvrant la première couche interne et étant composée de fibres coupées, dont au moins 25% en volume sont des fibres coupées hydrophiles; et
(c) des première et seconde couches externes de non tissés thermoplastiques positionnées sur les côtés opposés des couches internes recouvertes, lesdits non tissés ayant une taille moyenne de fibre ou de filament de 0,5 à 30 microns,
lesdites couches étant collées ensemble par des adhésifs placés aux interfaces des couches.

2. Stratifié tel que revendiqué dans la revendication 1, dans lequel la deuxième couche interne comprend au moins 25% en poids de fibres de coton.

3. Stratifié tel que revendiqué dans la revendication 2, dans lequel les fibres de coton sont sélectionnées à partir du groupe consistant en fibres cardées, fibres collées au latex et fibres collées thermiquement.

4. Stratifié tel que revendiqué dans la revendication 2 ou 3, dans lequel la deuxième couche interne comprend au moins 50% en poids de fibres de coton.

5. Stratifié tel que revendiqué dans la revendication 4, dans lequel la deuxième couche interne comprend 100% de fibres de coton.

6. Stratifié tel que revendiqué dans l'une quelconque des revendications précédentes, dans lequel la deuxième couche non tissée est une couche obtenue par soufflage-fusion ayant une taille moyenne de fibre de 0,5 à 15 microns.

7. Stratifié tel que revendiqué dans l'une quelconque des revendications précédentes, dans lequel les deux couches externes non tissées sont composées de non tissés obtenus par soufflage-fusion ayant un diamètre moyen de fibre de 0,5 à 15 microns.

8. Stratifié tel que revendiqué dans l'une quelconque des revendications précédentes, dans lequel le film microporeux a une grosseur moyenne de pore de 1,0 à 10 microns.

9. Stratifié tel que revendiqué dans l'une quelconque des revendications précédentes, dans lequel le stratifié a une épaisseur de 0,2 à 1,5 mil et a les propriétés suivantes: indice de transmission de vapeur d'au moins 400 g/m² par 24 heures et une résistance à l'éclatement d'au moins 15 psi.

10. Stratifié tel que revendiqué dans l'une quelconque des revendications précédentes, dans lequel le stratifié est capable de passer le Test de Résistance à la Pénétration du Sang de ASTM ES 21-92.

11. Stratifié tel que revendiqué dans l'une quelconque des revendications précédentes, dans lequel le stratifié est capable de passer le Test de Résistance aux Virus de ASTM ES 22-92.

12. Stratifié tel que revendiqué dans l'une quelconque des revendications précédentes, dans lequel le film microporeux est une polyoléfine remplie de particules étirée pour fournir une perméabilité à l'intérieur de celle-ci.

13. Stratifié tel que revendiqué dans l'une quelconque des revendications précédentes, dans lequel le film microporeux représente au moins 25% en poids du stratifié.

14. Stratifié tel que revendiqué dans l'une quelconque des revendications précédentes, dans lequel l'adhésif est un adhésif à fusion à chaud.

15. Stratifié tel que revendiqué dans la revendication 14, dans lequel l'adhésif collant les couches ensemble fait de 1 à 10 grammes/m².

16. Stratifié tel que revendiqué dans la revendication 14 ou 15, dans lequel l'adhésif est en la forme de couches qui ont été pulvérisées sur les surfaces du stratifié et ensuite collées sous pression à une température de moins de 100°C.

17. Stratifié convenant aux emplois médicaux qui comprend:
(a) un premier non tissé sélectionné à partir du groupe consistant en non tissés thermoplastiques obtenus par filage-liaison et soufflage-fusion et ayant une surface externe et une surface interne;
(b) un film microporeux ayant une surface externe faisant face au premier non tissé et adhésivement collée à celui-ci, et une surface faisant face à l'intérieur;
(c) un tissu en fibres coupées comprenant au moins 25% en poids de fibres hydrophiles, et ayant une surface externe adhésivement collée à la surface faisant face à l'intérieur du film microporeux, et une surface faisant face à l'intérieur;
(d) un deuxième non tissé sélectionné à partir du groupe consistant en non tissé thermoplastique obtenu par soufflage-fusion à partir de non tissés obtenus par filage-liaison et soufflage-fusion, et ayant une surface externe adhésivement collée à la surface faisant face à l'intérieur du tissu en fibres coupées.

18. Stratifié tel que revendiqué dans la revendication 17, dans lequel le film microporeux est composé de films sélectionnés à partir du groupe consistant en polyoléfines, polyamides, polyuréthanes, et PTFE.

19. Stratifié tel que revendiqué dans la revendication 17 ou 18, dans lequel le tissu de fibres coupées comprend plus de 50% en poids de fibres de coton.

20. Stratifié tel que revendiqué dans l'une quelconque des revendications 17 à 19, dans lequel le film microporeux est préparé en étirant une polyoléfine ayant un garnissage inorganique dispersé sur celle-ci pour donner une porosité suffisante à celle-ci afin de fournir au film une aptitude respiratoire.

21. Méthode de fabrication d'un stratifié comprenant:
(a) sélectionner une couche d'un premier non tissé et une couche de film microporeux;
(b) pulvériser un adhésif fondu sur une surface soit de non tissé soit de film microporeux;
(c) superposer le premier non tissé et le film microporeux avec l'adhésif pulvérisé dessus entre ceux-ci, le film microporeux ayant une surface interne faisant face au premier non tissé et une surface externe;
(d) sélectionner une couche d'un deuxième non tissé et une couche de fibres coupées;
(e) pulvériser un adhésif fondu soit sur la surface du deuxième non tissé soit sur la couche de fibres coupées;
(f) superposer le deuxième non tissé et la couche de fibres coupées avec l'adhésif pulvérisé dessus entre ceux-ci, ladite couche de fibres coupées ayant une surface interne faisant face au deuxième non tissé et une surface externe;
(g) pulvériser un adhésif soit sur la surface externe du film microporeux soit sur le deuxième non tissé;
(h) amener les quatre couches selon un rapport de superposition à travers la ligne de contact de rouleaux de calandre pressurisés à rotation antagoniste dans lesquelles l'adhésif est à l'interface de chacune des couches, en vertu de quoi les couches sont collées ensemble sous pression par les adhésifs.

22. Méthode telle que revendiquée dans la revendication 21, dans laquelle les fibres coupées comprennent plus de 50% en poids de fibres de coton.

23. Méthode telle que revendiquée dans la revendication 21 ou 22, dans laquelle le premier non tissé est sélectionné à partir du groupe consistant en non tissés obtenus par soufflage-fusion et filage-laison et le deuxième non tissé est un non tissé obtenu par soufflage-fusion.

24. Méthode telle que revendiquée dans l'une quelconque des revendications 21 à 23, dans laquelle la pression est de 10 à 200 psi.

25. Méthode telle que revendiquée dans l'une quelconque des revendications 21 à 24, dans laquelle la surface des rouleaux presseurs est faite en un matériau élastique lisse.

26. Méthode telle que revendiquée dans l'une quelconque des revendications 21 à 25, dans laquelle la surface des deux rouleaux est lisse.

27. Méthode telle que revendiquée dans l'une quelconque des revendications 21 à 26, dans laquelle l'une des surfaces des rouleaux de calandre est gaufrée.

28. Méthode telle que revendiquée dans l'une quelconque des revendications 21 à 27, dans laquelle l'adhésif est un adhésif à fusion à chaud.

29. Méthode telle que revendiquée dans l'une quelconque des revendications 21 à 28, dans laquelle la température des rouleaux est maintenue à moins de 100°C.

30. Vêtement de protection pour couvrir un membre du corps afin de protéger le membre du corps d'être en contact avec du sang ou des liquides étrangers qui comprend
(a) une première couche thermoplastique non tissée ayant une surface externe étant disposée vers l'extérieur du membre du corps, et une surface interne faisant face au membre du corps;
(b) une couche de fibres coupées ayant une surface externe faisant face vers l'extérieur du membre du corps, et une surface interne faisant face à la première couche non tissée et étant adhésivement collée à la surface externe de la première couche non tissée;
(c) un film microporeux ayant une surface externe faisant face vers l'extérieur du membre du corps et une surface interne faisant face à la surface externe de la couche de fibres coupées et étant adhésivement collée à celle-ci;
(d) une deuxième couche thermoplastique non tissée ayant une surface externe faisant face vers l'extérieur du membre du corps et une surface interne adhésivement collée à la surface externe de la couche de film microporeux;

31. Vêtement tel que revendiqué dans la revendication 30, dans lequel la couche de fibres coupées inclut des fibres coupées de coton et la deuxième couche non tissée comprend un tissu obtenu par soufflage-fusion.

32. Stratifié comprenant une couche centrale de fibres coupées de coton et des couches flanquantes d'un film microporeux et d'une couche non tissée sélectionnées à partir du groupe consistant en fibres ou filaments thermoplastiques traités par soufflage-fusion ou filage-liaison, lesdites couches flanquantes étant adhésivement collées aux surfaces opposées de la couche centrale.
